Europäisches Patentamt

European Patent Office

Office européen des brevets

(19)

(11) Publication number : **0 524 718 A1**

(12) **EUROPEAN PATENT APPLICATION**

(21) Application number : 92304602.3

(22) Date of filing : 20.05.92

(51) Int. Cl.$^5$ : **C08G 18/08, C08G 18/10, A61K 9/00, C08G 18/76**

(30) Priority : **24.05.91 US 705730**

(43) Date of publication of application :
**27.01.93 Bulletin 93/04**

(84) Designated Contracting States :
**AT BE CH DE DK ES FR GB GR IT LI LU MC NL PT SE**

(71) Applicant : **GENSIA PHARMACEUTICALS, INC.**
**11025 Roselle Street**
**San Diego, CA 92121 (US)**

(72) Inventor : **Hahn, soonkap**
**17110 Cliquot Court**
**Poway, California 92064 (US)**

(74) Representative : **Sexton, Jane Helen et al**
**J.A. Kemp & Co. 14 South Square Gray's Inn**
**London WC1R 5LX (GB)**

(54) Polyurethane hydrogel compositions for iontophoretic drug delivery.

(57)  Methods of preparing hydrogel matrices having a high swelling ratio, good tensile strength and optical transparency which are useful for transfermal iontophoretic delivery of drugs are provided. Also provided are hydrogel matrices prepared according to those methods.

FIGURE 1

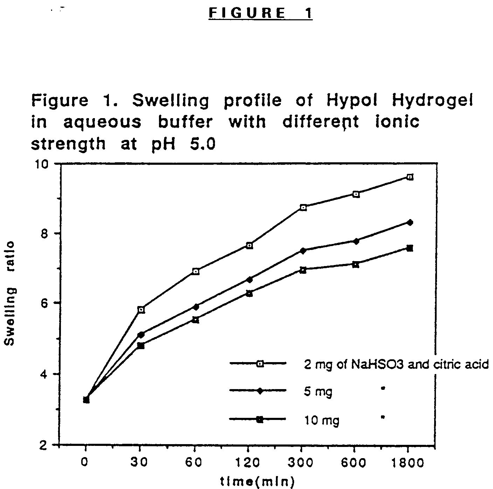

Figure 1. Swelling profile of Hypol Hydrogel in aqueous buffer with different ionic strength at pH 5.0

Jouve, 18, rue Saint-Denis, 75001 PARIS

The present invention is directed to polyurethane hydrogel matrices which are suitable compositions for in vivo iontophoretic drug delivery and for methods for preparing these polyurethane hydrogel compositions.

These hydrogel compositions may be used as monolithic drug reservoirs for the transdermal iontophoretic delivery of drugs. These compositions alternatively may be used as passive transdermal drug reservoirs. An additional utility is as transparent hydrophilic wound dressings.

Iontophoresis is the transport of ionized or charged species by application of an electrical current. Transdermal iontophoresis is the transport of an ionic drug through the skin of a mammal, usually a human, by passing a current through a drug containing electrode placed against the skin. A second electrode, termed the return or indifferent electrode, is placed against the skin, normally several inches from the first. The current is evoked by applying a potential between the electrodes in a constant DC., pulsed DC or AC mode. The current carries the ionized drug "through" the stratum corneum into the dermis where the drug diffuses into the capillaries situated near the dermal-epidermal junction, and then into the systemic circulation.

Although the present invention has general application in virtually all circumstances where a drug is administered iontophoretically, the primary intended uses for the hydrogel matrices of the present invention are in conjunction with administration of certain exercise simulating agent (ESA) drugs on human patients, as described in several commonly assigned and co-pending patent applications. For this reason additional background will be provided concerning these exercise simulating agent drugs and the delivery systems, including the electrodes, preferably used.

Exercise simulating agents are drugs that elicit acute and adaptive cardiovascular responses similar to the types of responses elicited by aerobic activity. They are particularly useful, therefore, as a substitute for exercise stress testing for diagnosing cardiovascular diseases, due to their ability to increase heart rate, myocardial contractility, arterial blood pressure, and coronary and skeletal muscle blood flow. Exercise simulating agents of sufficient potency and iontophoretic mobility can be advantageously delivered, i.e. transported through the stratum corneum, using iontophoresis.

The amount of drug transported into the skin per unit time per unit area is known as the flux. Iontophoretic flux is proportional to the applied electrical potential and the drug concentration on the outside of the skin. The upper limit of current density to ensure that the current will not damage the skin is generally considered to be 0.5 mAmp/cm$^2$ of DC current. Other limits on flux include drug solubility, the partition coefficient of drug in the stratum corneum, and the drug's iontophoretic mobility. Clinically effective transdermal iontophoresis must be achieved within these limits on the flux.

In addition to the above-listed factors, the flux is greatly affected by several other factors. For example, the drug's ability to pass freely, i.e., its mobility, often depends on the pH of the solution in which it is delivered, and optimum iontophoretic mobility requires that the drug be ionized or in charged form at some specific pH. Also, reduced iontophoretic efficiency, i.e., lower flux of a given drug, can result from the presence of other ionic species in the formulation. These other ions will "compete" with the drug ions as current carrier and can drastically reduce iontophoretic flux.

Several types of electrode pads have been proposed for use in transdermal iontophoresis. These may be classified as: (1) monolithic pad; (2) reservoir pad; and (3) multilayer pad.

A monolithic electrode pad design provides for including the drug in a polymer or matrix that is attached to the electrode. The polymer may contain an adhesive to maintain contact with the patient's skin. The drug is dispersed in the polymer during manufacture and the polymer is then formed into the pad itself. Due to their ease of manufacture, simplicity of construction and predictability of drug transport phenomena, electrode pads of monolithic design have been proposed as a preferred design for iontophoretic drug delivery.

Monolithic gel pad designs using other gel forming substances have limitation. For example, those made of methocel (hydroxypropylmethylcellulose) have proved to exhibit flowability, being too flowable during pad application and removal, and have proved difficult to store. Gel formulations comprising polyhydroxy ethyl methacrylate (poly HEMA) have exhibited an insufficient swelling ratio.

## SUMMARY OF THE INVENTION

The present invention is directed to methods of preparing a polyurethane hydrogel matrix suitable for use in transdermal iontophoretic drug delivery and to the hydrogel matrices produced thereby. These hydrogel matrices are particularly useful for iontophoretic delivery of exercise simulating β-agonists, including certain catecholamine derivatives and analogues.

The hydrogel matrices of the present invention preferably exhibit (a) a high swelling ratio in the conducting medium; (b) good tensile strength; and (c) optical transparency. A high swelling ratio provides high iontophoretic efficiency and compliance characteristics. Compliance refers to flexibility of the gel matrix, that is, the ability of the gel matrix to conform to an irregular surface. Good tensile strength allows for ease of handling of the gel matrix during manufacture. However, if the gel is too hard (i.e. overly cross-linked), it may exhibit insufficient compliance due to a low swelling ratio. Optical transparency indicates an absence of entrapped bubbles or other irregularities in the gel matrix and the absence of precipitated prepolymer.

According to one aspect, the present invention provides a method of preparing a polyurethane hydrogel matrix. According to one embodiment, this method comprises first dissolving an isocyanate-capped oxyalkylene-based prepolymer in a first solvent which comprises an anhydrous aprotic organic solvent to give a prepolymer solution. The prepolymer solution is mixed with a second solvent which comprises water and optionally a water-miscible organic solvent to give a hydrogel forming mixture. The water-miscible organic solvent may be protic or aprotic. The hydrogel forming mixture is allowed to cure to give a hydrogel matrix. After evaporation of organic solvent, the hydrogel matrix may then be loaded with drug by swelling in an appropriate swelling medium. The ratio of prepolymer to water used according to this embodiment of the present invention is such that the isocyanate (NCO) content of the prepolymer used to volume of water is preferably from about 0.04 meq isocyanate per ml water to about 4 meq isocyanate per ml water, and the ratio of prepolymer to total solvent volume (volumes of first solvent and second solvent) is such that the isocyanate content of the prepolymer used is from about 0.01 meq isocyanate per ml total solvent to about 1 meq isocyanate per ml total solvent.

According to an alternate embodiment of the present invention, the method of preparing the hydrogel matrices comprises mixing an isocyanate-capped oxyalkylene-based prepolymer in a total solvent which comprises water and a water-miscible organic solvent to give a hydrogel-forming mixture. The water miscible organic solvent may be protic or aprotic. The hydrogel forming mixture is allowed to cure to give a hydrogel matrix. After evaporation of organic solvent, the hydrogel matrix may be loaded with drug by swelling in an appropriate swelling medium. The ratio of prepolymer to water used according to this embodiment in such that the isocyanate (NCO) content of the prepolymer used to volume of water is preferably from about 0.04 meq isocyanate per ml water to about 4 meq isocyanate per ml water, and the ratio of prepolymer to total solvent is from about 0.01 meq per ml total solvent to about 1 meq isocyanate per ml total solvent. Preferably the ratio of water to water-miscible organic solvent is about 0.2 parts to about 1 parts water per part water miscible organic solvent.

The present invention is also directed to Hydrogel matrices prepared by these methods.

## BRIEF DESCRIPTION OF THE DRAWINGS

FIG. 1 depicts the swelling profile of hydrogel matrices prepared using Hypol Hydrogel prepolymers in aqueous buffer with different ionic strength at pH 5.0.

FIG. 2 depicts the swelling profiles of hydrogel matrices prepared using certain prepolymers from the Hypol® series.

FIG. 3 depicts the drug loading profile of a hydrogel matrix of the present invention prepared using Hypol Hydrogel prepolymer.

FIG. 4 depicts equilibrium drug loading profiles of hydrogel matrices of the present invention prepared using certain prepolymers from the Hypol series in solution at different drug concentrations.

FIG. 5 depicts drug releasing profiles of hydrogel matrices of the present invention prepared using certain prepolymers from the Hypol series.

FIG. 6 is a plot of drug flux through porcine epidermis versus current density from a hydrogel matrix of the present invention prepared using Hypol Hydrogel prepolymer loaded with an exercise simultating agent.

FIG. 7 depicts flux at two drug concentrations as a function of current density.

FIG. 8 depicts flux at various current densities as a function of drug concentration.

FIG. 9 depicts maximum heart rate change versus drug concentration in a hydrogel matrix prepared using Hypol Hydrogel prepolymer.

FIG. 10 depicts the swelling behavior of a hydrogel matrix prepared using Hypol Hydrogel prepolymer at different temperatures.

FIG. 11 depicts the temperature dependency of the swelling ratio of a hydrogel matrix of the present invention prepared using Hypol Hydrogel which was swollen in two different solutions.

## DETAILED DESCRIPTION OF THE INVENTION

### Preferred Polyurethane Hydrogel Compositions

According to the present invention, preferred polyurethane hydrogel matrices are provided that are suitable for use in transdermal iontophoretic delivery of drugs. These hydrogel matrices advantageously exhibit high iontophoretic efficiency, good tensile strength and swelling behavior, and optical transparency. These hydrogel matrices do not have the bubble foam structure which normally results from carbon dioxide production during the cross-linking reaction between the isocyanate (-NCO) groups of the prepolymer and the hydroxyl groups of water. The absence of vacancies such as bubbles and other imperfections in the gel matrix allows for continuity of electrical current and thus high iontophoretic efficiency. Electrode pads comprising these hydrogel matrices have demonstrated comparable iontophoretic drug delivery as compared with electrode pads prepared using cellulosic gel-forming materials such as hydroxypropylmethylcellulose (Methocel®). (See Examples 14 and 15 and Tables X and XI).

These gel matrices exhibit both good tensile strength and swelling behavior. The tensile strength of the gel matrix is related to its extent of cross-linking. Insufficient cross-linking results in a hydrogel which is too flowable and soft and, thus, is unsuitable for use as an electrode pad. However, overly crosslinked hydrogel matrices possess inferior swelling behavior. Swelling behavior of the hydrogel matrix reflects its mechanical properties (such as compliance), iontophoretic efficiency, and ability to load drug. The rate and extent of swelling of the hydrogel in aqueous solution is related to its hydrophilicity, cross-link density, temperature, and, if loaded with charged (ionic) moieties (as for iontophoretic delivery of a drug), the ionic composition of the solvent and/or pH.

In view of their combination of good tensile strength coupled with good swelling behavior, preferred are hydrogel compositions prepared using a prepolymer content of at least about 5% for prepolymer having an isocyanate content of about 0.4 meq/g. However, to insure that the resulting gel matrix is not overly crosslinked, it is preferred to use no more than about 25% of prepolymer having an isocyanate content of about 0.4 meq/g. Where prepolymers having different isocyanate contents are employed, the amount of prepolymer used should be adjusted accordingly. For example, it is preferred to use at least 7.5% but less than 25% of prepolymer having an isocyanate content of about 0.8 meq/g. It is also preferred to use at least 10% but less than 25% of prepolymer having an isocyanate content of about 1.6 meq/g.

Preferred are hydrogel matrices having a tensile strength of about 5 psi to about 25 psi and having a swelling ratio of at least about 4, preferably from about 7 to about 15.

These hydrogel matrices also advantageously exhibit optical transparency. Opacity of the matrix results not only from bubble entrapment during the crosslinking reaction, but also may result from precipitation of prepolymer in the reaction mixture due to low solubility of the prepolymer in water. We have found that once opacity is attained (see Example 3 and Table III), such opacity is not reversible to transparency even after the gel matrix is fully swollen. We found that hydrogel matrices prepared using about 50% acetone in total solvent showed very little foam character (i.e. bubble entrapment), but some opacity. Transparency of the gel matrix is desirable, since it allows easy observation of any discoloration after drug is loaded in the matrix.

## Preferred Prepolymers

Suitable prepolymers for use in preparing the hydrogel matrices of the present invention are described in U.S. Patent No. 4,940,737 to Braatz et al., the disclosure of which is incorporated by reference herein.

One group of isocyanate-capped urethane prepolymers of this class that can be used in the methods and hydrogel matrices of the present invention comprises isocyanate-capped polyesters. Such prepolymers may be made by condensing a polyhydric alcohol with a polycarboxylic acid to form a linear polyester which is then reacted with a slight molar excess of a polyisocyanate to provide an essentially linear polyurethane having terminal isocyanate groups and having an average molecular weight within the range 100 to 20,000, preferably between about 600 to about 6000. Polyhydric alcohols that can be used in preparing such prepolymers include polyalkylene glycols such as ethylene, propylene and butylene glycol and polymethylene glycols such as tetramethylene and hexamethylene glycols. Another group of isocyanate capped urethane prepolymers that can be used comprise isocyanate capped polyethers. These prepolymers can be made by reacting, for example, polyalkylene glycols with suitable diisocyanates (which include aromatic, aliphatic or cycloaliphatic di- or polyfunctional isocyanates) to provide a polyurethane having terminal isocyanate groups and having an average molecular weight within the range 100 to 20,0000, preferably between about 600 to about 6000. As specific examples of these prepolymers, the HYPOL™ polyurethane prepolymer series available from Grace Speciality Chemicals Co., W.R. Grace & Co. Conn., is suitable.

A second class of prepolymers suitable for use in this invention comprises polyoxyalkylene diols or polyols which are of generally higher molecular weights and which are predominantly or exclusively made up of ethylene oxide monomer units. Preferably, at least 75% of the monomer units should be ethylene oxide, more preferably at least 90%, and most preferably at least 95% up to about 100%. As specific examples of this class of prepolymers, prepolymers from the BIOPOL™ polyurethane prepolymer series available from grace Speciality Chemicals Co., W.R. Grace & Co. Conn., will be particularly suitable.

High molecular weight ethylene oxide-based diols and polyols are used to prepare this second class of prepolymers. The diol or polyol molecular weight prior to capping with polyisocyanate preferably should be at least about 7000 to 8000 MW, more preferably about 10,000 to about 30,000 MW. It is preferred to use trihydroxy compounds (triols) in the preparation of the polyols which are the precursors to the prepolymers. For example, glycerol is a preferred triol. Trimethylolpropane (TMOP), trimethylolethane and triethanolamine are other suitable triols. In addition, tetrols, such as pentaerythritol, may be used to prepare polyols for use in this invention. Triol- or tetrol-based polyols are capped with difunctional or polyfunctional isocyanate compounds to form the prepolymer.

Preferred prepolymers include certain isocyanate-capped oxyethylene based prepolymers available from W.R. Grace & Co. These include those available under the names Biopol™ (having an isocyanate content of about 0.4 meq/g), Hypol® Hydrogel (having an isocyanate content of about 0.8 meq/g), Hypol® 2002 (having an isocyanate content of about 1.6 meq/g).

Preparation of Preferred Hydrogel Matrices

The present invention provides methods for the preparation of hydrogel matrices which are highly swellable (in aqueous solution), yet have good tensile strength, are optically transparent and exhibit high iontophoretic efficiency, but do not exhibit the bubble foam structure of matrices conventionally formed during the crosslinking reaction using hydrophilic polyurethane prepolymers. As noted above, suitable prepolymers include those available under the trademarks Hypol® 2002, Hypol® Hydrogel and Biopol™ from W.R. Grace & Co.

According to one embodiment, the prepolymer is dissolved in a substantially dry aprotic water miscible organic solvent to give a prepolymer solution. Preferably solvent is added in the amount of about 0.2 to about 0.5 parts prepolymer per part solvent. Preferably sufficient solvent is added to give a prepolymer solution of about 20% to about 40% prepolymer in solvent. Suitable aprotic, water miscible organic solvents include acetone, methyl ethyl ketone, dimethylformamide/dimethylsulfoxide and the like. To the prepolymer solution, a second solvent is added which comprises water and optionally a water miscible organic solvent to give a hydrogel forming solution. The water miscible organic solvent may be protic or aprotic. Suitable water miscible organic solvents include acetone, ethanol, propylene glycol, isopropyl alcohol and the like. Water is added in a sufficient amount to the prepolymer solution such that the isocyanate content of prepolymer per ml water is from about 0.04 to about 4 meq isocyanate per ml water. Particularly preferred ratios of isocyanate content to water are those which comprise from about 0.04 to about 0.32 meq isocyanate per ml water. The second solvent is added in sufficient amount to give a hydrogel forming solution which comprises (before cross-linking) from about 5% to about 25% prepolymer, preferably from about 5% to about 10% prepolymer. Preferably the second solvent comprises from about 0.5 parts to about 1 part water to about 1 part water miscible solvent. The gel matrix is allowed to cure. Preferably, organic solvent is removed from the hydrogel matrix by evaporation prior to swelling.

An alternate embodiment of the present invention provides a method for preparing the hydrogel matrices which comprises a single mixing step. According to this embodiment, prepolymer is mixed with the total solvent which comprises water and water-miscible organic solvent to give a hydrogel forming solution. Suitable water miscible organic solvents include acetone, methyl ethyl ketone, dimethylformamide/dimethylsulfoxide and the like. Water is added in a sufficient amount (as part of total solvent) that the isocyanate content of prepolymer per ml water is from about 0.04 meq to about 4 meq isocyanate per ml water. Particularly preferred ratios of isocyanate content to water are those which comprise from about 0.04 meq to about 0.32 meq isocyanate per ml water. Total solvent is added in a sufficient amount to give a hydrogel forming solution which comprises (before cross-linking) from about 5% to about 25% prepolymer, preferably from about 5% to about 10% prepolymer. Preferably total solvent comprises about 0.2 to about 1 part water per part water miscible organic solvent. The gel matrix is allowed to cure and organic solvent is preferably removed, by methods such as evaporation, before swelling of the hydrogel matrix.

In the crosslinking reaction, an isocyanate group of the prepolymer reacts with an hydroxyl of water to give an amino group and generate carbon dioxide; the amino group then reacts with another isocyanate group of a prepolymer to give a urea cross-link. Accordingly, the ratio of isocyanate groups,to hydroxyl groups and the overall concentrations of prepolymer, first solvent and second solvent or total solvent are important in obtaining a hydrogel matrix without a foam structure (from entrapped carbon dioxide) and which is neither overly nor insufficiently crosslinked. Examples 1, 2, 3 and 4 demonstrate the preparation of hydrogel matrices of the present invention, and also demonstrate the importance of controlling the ratio of prepolymer and isocyanate groups to water and the overall concentration of prepolymer in solution.

Swelling of Hydrogel Matrix and Drug Loading

The swelling ability of the hydrogel matrices of the present invention allows separation of the polymerization/ crosslinking process from the drug loading process and also gives an opportunity to remove residual impurities from the hydrogel matrix after polymerization and before drug loading.

Swelling behavior of these gel matrices is sensitive to changes in swelling medium conditions such as ionic strength, pH, organic solvent presence and temperature.

When deionized water is used as the swelling medium, the equilibrium swelling ratios of hydrogel matrices of the present invention made using Hypol 2002 prepolymer are about 2.5 to about 4, those made using Hypol Hydrogel are about 7 to about 10 and those made using Biopol prepolymer are about 10 to about 20.

These hydrogel matrices may be conveniently loaded with drug for transdermal administration by swelling the hydrogel matrix in an aqueous solution of drug.

In one embodiment, the hydrogel matrix was swollen using an aqueous solution of drug containing a chemical enhancer for enhancing transdermal penetration of drug. One suitable enhancer is ethanol. Other suitable enhancers include propylene glycol and glycerol. Suitable ethanol-containing swelling media comprise from about 30% to about 50% ethanol, more preferably from about 40% to about 50% ethanol.

In another embodiment of the present invention, these hydrogel matrices are swollen with an aqueous drug solution comprising from about 30% to about 50% propylene glycol. We have found that use of these propylene glycol-containing swelling media have a humectant effect and, thus, retard dehydration of the hydrogel matrix.

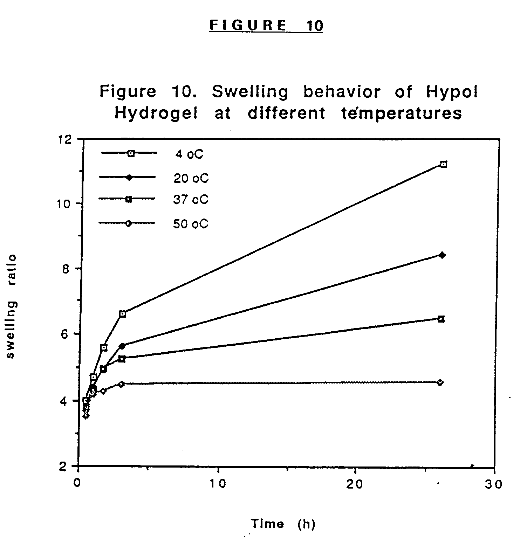

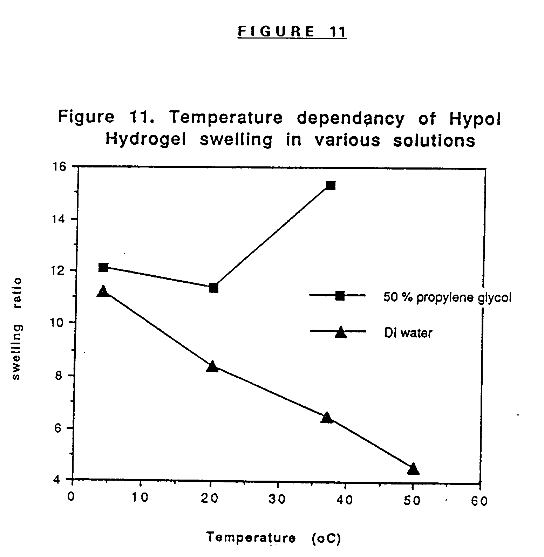

We have found that the sensitivity of swelling behavior of these hydrogel matrices makes them subject to dimensional changes (syneresis) on long term storage and upon change in temperature (see Figure 11). We have found that a hydrogel matrix swollen at one temperature may show syneresis when it is stored at a different, higher temperature. As shown in Figure 11, if a hydrogel matrix is swollen at a lower temperature than the anticipated storage temperature, the volume of the swollen hydrogel matrix will decrease at the higher temperature thereby squeezing some of the swelling medium (including drug) out. Thus, in order to decrease syneresis, these hydrogel matrices may be swollen at a higher temperature than the anticipated storage temperature or swollen incompletely. In the incomplete swelling method, the gel would be swollen for only 2 to 3 hours such that it has not reached equilibrium swelling for that temperature (See Fig. 10). Both of these methods would result in a hydrogel matrix which was swollen to a slightly lower extent than that of equilibrium swelling at the anticipated high limit of storage temperature. Therefore, the hydrogel matrices swollen under either of these sets of conditions would show decreased syneresis phenomena on temperature change. A typical storage temperature is at least 10°C, commonly about 20 to 25°C. Alternatively, we have found that hydrogel matrices swollen in aqueous swelling media comprising propylene glycol, as described above, exhibit decreased syneresis with temperature changes. Temperature dependency of a hydrogel matrix prepared using Hypol Hydrogel prepolymer swollen in deionized water and 50% propylene glycol is shown in Figure 11.

To assist in understanding the present invention, the following examples are included which describe the results of a series of experiments. The following examples relating to this invention should not, of course, be construed in specifically limiting the invention and such variations of the invention, now known or later developed, which would be within the purview of one skilled in the art are considered to fall within the scope of the present invention as hereinafter claimed.

EXAMPLES

EXAMPLE 1

Preparation of Hydrogel Matrices (I)

Hypol® hydrogel prepolymer (W.R. Grace & Co.), 0.4 g was dissolved in 1 ml acetone in a beaker. To this solution, 3 ml of acetone:water (2 ml acetone:1 ml water) was added, and stirred manually to mix. The resulting mixture was allowed to stand covered (to avoid excessive solvent evaporation). After curing was complete (about 20 minutes), the gel was released from the container. The gel was placed in a hood overnight to evaporate acetone.

Additional hydrogel matrices were prepared as described above and are summarized in Table I, using the noted amounts of prepolymer and solvent.

TABLE I

Preparation of Gels Using Hypol Hydrogel™

| Gel No. | Wt. Prepolymer | Solvent 1 | Solvent 2 | Gel Properties |
|---------|----------------|-----------|-----------|----------------|
| I-A | 1.0 g | 1 ml acetone | 0.5 ml water/1.5 ml acetone | too hard |
| I-B | 0.5 g | 1 ml acetone | 0.5 ml water/1.5 ml acetone | hard |
| I-C | 0.3 g | 1 ml acetone | 0.5 ml water/1.5 ml acetone | good gel |
| I-D | 0.15 g | 1 ml acetone | 0.5 ml water/1.5 ml acetone | too soft |
| I-E | 0.3 g | 1 ml acetone | 1 ml water/2 ml acetone | good gel |
| I-F | 0.2 g | 1 ml acetone | 1 ml water/2 ml acetone | soft |

EXAMPLE 2

Preparation of Hydrogel Matrices (II)

Gel formulations were prepared using the below-noted amounts of Biopol™ (W.R. Grace & Co.) prepolymer and the following amounts of solvents according to the following protocol:

Prepolymer was solubilized in solvent 1. Solvent 2 was added. The resulting mixture was cured and had the noted properties. Hydrogel matrices prepared according to the above protocol are summarized in Table II.

## EXAMPLE 3

### Preparation of Hydrogel Matrices (III)

Hydrogel matrices were prepared using Hypol® 2002 prepolymer (W.R. Grace & Co.) in the following

### TABLE II

### Preparation of Gels Using Biopol™

| Gel No. | Wt. Prepolymer | Solvent 1 | Solvent 2 | Gel Properties |
|---|---|---|---|---|
| II-A | 0.3 g | 1 ml acetone | 2 ml acetone/1 ml water | cured overnight |
| II-B | 0.3 g | 1 ml acetone | 1 ml acetone/1 ml water | cured overnight, good gel |
| II-C | 0.3 g | 1 ml acetone | 1 ml acetone/2 ml water | cured after 2 hours, some opacity |
| II-D | 0.3 g | 2 ml acetone | 2 ml acetone/2 ml water | nice gel |
| II-E | 0.1 g | 2 ml acetone | 1 ml acetone/1 ml water | soft |
| II-F | 1.0 g | 2 ml acetone | 1 ml acetone/1 ml water | too hard |
| II-G | 0.4 g | none | 4 ml water | opaque with some bubbles |

amounts with the following amounts of the noted solvents according to the following protocol.

Prepolymer was solubilized in Solvent 1 by manual mixing. Solvent 2 was added with manual mixing. The resulting mixture was allowed to cure. The properties of the resulting gel were observed.

Hydrogel matrices so prepared according to the above protocol are summarized in Table III.

TABLE III

(Hypol® 2002)                    Preparation of Gels with Hypol® 2002

| No. | Prepolymer | Solvent 1 | Solvent 2 | Properties of Gel |
|---|---|---|---|---|
| | 1.2 g | 1 ml acetone | 1.0 ml acetone: 0.5 ml water | too hard gel |
| | 1.2 g | 1 ml acetone | 1.0 ml acetone: 1 ml water | too hard gel |
| | 1 g | 1 ml ethanol/1 ml water | ---- | opaque |
| | 1 g | 1 ml ethanol | 1 ml ethanol/1 ml water | did not cure |
| | 1 g | 1 ml isopropanol | 1 ml ethanol/ 1 ml water additional 0.5 ml water after 30 min. | did not cure |
| | 1 g | 0.5 ml ethanol/1 ml water | 1.5 ml ethanol/water | did not cure |
| | 1 g | 0.5 ml acetone | 0.75 ml ethanol/0.75 ml water | did not cure well enough |
| | 1 g | 0.5 ml acetone | 0.5 ml ethanol/1 ml water | cured with some opacity |
| | 1 g | 1 ml acetone | 0.5 ml ethanol/1 ml water | transparent gel which turned opaque in water |

EP 0 524 718 A1

TABLE III (Continued)

| Gel No. | (Hypol® 2002) Prepolymer | Preparation of Gels with Hypol® 2002 | | |
|---|---|---|---|---|
| | | Solvent 1 | Solvent 2 | Properties of Gel |
| III-J | 1 g | 1 ml acetone | 1 ml ethanol/1.5 ml water | did not cure |
| III-K | 1 g | 1 ml PEG (Dow, 400 N.F.) 30 minutes | 0.5 ml water | $CO_2$ bubbling |
| III-L | 1 g | 1 ml PEG (Dow, 400 N.F.) | 0.5 ml ethanol/0.5 ml water | too flowable, did not cure |
| III-M | 1 g | 1 ml PEG (Dow, 300 N.F.) | 0.5 ml ethanol/0.5 ml water | too flowable |
| III-N | 0.3 g | 1 ml acetone | 0.5 ml acetone/0.5 ml water | hard |
| III-O | 0.3 g | 1 ml acetone | 1 ml acetone/1 ml water | nice gel, with slight opacity |
| III-P | 0.3 g | 1 ml acetone | 2 ml acetone/1 ml water | too soft |
| III-Q | 0.4 g | 2 ml acetone | 2 ml acetone/2 ml water | too soft |

EXAMPLE 4

Preparation of Hydrogel Matrices (IV)

Hydrogel matrices of the present invention were prepared according to the following one-step protocol. These matrices are summarized in Table IV.

The noted amount of prepolymer was mixed with total solvent comprising water and water miscible organic solvent in the noted amounts. The resulting hydrogel matrix was allowed to cure. Excess solvent was removed by evaporation.

TABLE IV

Preparation of Hydrogel Matrices (One Step Protocol)

| Gel No. | Prepolymer, Amount | Total Solvent | Gel Properties |
|---------|--------------------|--------------|----------------|
| IV-A | Hypol Hydrogel, 0.3 g | 1 ml water/ 2 ml acetone | good gel, no opacity |
| IV-B | Hypol Hydrogel, 0.3 g | 1.5 ml water/ 1.5 ml acetone | good bubble control with opacity |
| IV-C | Hypol 2002, 0.3g | 1 ml water/ 2 ml acetone | good bubble control with opacity |
| IV-D | Hypol 2002, 0.3g | 0.5 ml water/ 2.5 ml acetone | good bubble control, transparent, a little soft |

EXAMPLE 5

Preparation of Hydrogel Matrix Using Hypol Hydrogel

Into a large glass crystallizing dish (PYREX® 125 x 6), 6 g of Hypol® Hydrogel prepolymer were placed. The prepolymer was dissolved in 30 ml acetone by stirring with a spatula. To that solution, 30 ml of a 1:1 mixture of deionized water:acetone was added and the resulting solution mixed well with a spatula. The dish was covered with aluminum foil for 30 minutes. The resulting matrix was then allowed to air dry with no cover for one hour. The hydrogel matrix was removed from the dish, invented, replaced in the dish and then allowed to air dry for at least 4-5 hours.

The resulting matrix may then be swelled using an appropriate swelling medium.

EXAMPLE 6

Preparation of Hydrogel Matrix Using Hypol 2002

Into a large glass crystallizing dish (PYREX® 125 x 65) 6 g Hypol® 2002 were placed. The prepolymer was dissolved in 25 ml acetone by stirring with a spatula. To this solution 25 ml of a 3:2 acetone (15 ml):deionized water (10 ml) mixture was added and the resulting solution mixed well with a spatula. The dish was covered with aluminum foil for 30 minutes. The resulting matrix was then allowed to air dry for one hour. The matrix was removed from the dish, inverted, replaced in the dish and allowed to air dry for at least 4 to 5 hours.

The resulting matrix may then be swelled using an appropriate swelling medium.

EXAMPLE 7

Swelling Behavior of Hydrogel Matrices

Swelling behavior of a hydrogel matrix may be monitored by immersing a partially or completely desiccated hydrogel matrix in aqueous solvent and then removing it and weighing it as a function of time. Both the rate of solvent uptake and the plateau value for the ratio of swollen to initial weight may then be compared among formulations or as a function of ionic strength.

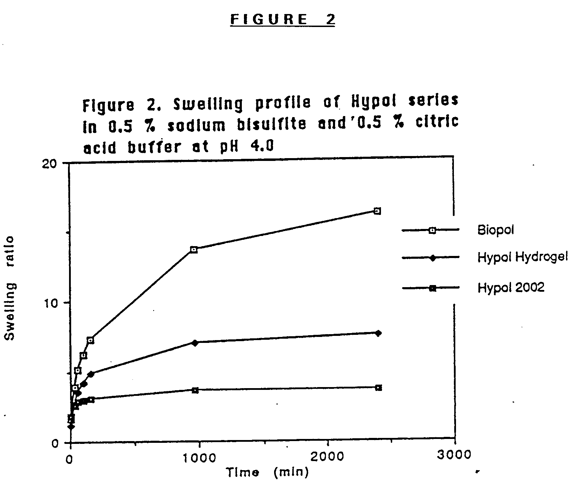

Swelling behaviors of hydrogel matrices prepared from the following prepolymers: Hypol® 2002, Hypol® Hydrogel and Biopol™ (all obtained from W.R. Grace & Co.) were investigated using aqueous solutions with different ionic strengths. Since these matrices may possess a slight polyelectrolytic character, different swelling behavior was expected upon changing the ionic strength of the solution. Figure 1 depicts the swelling behavior of a hydrogel matrix prepared using Hypol Hydrogel prepolymer and following the typical process described in Example 5 at several different ionic strengths. Swelling behavior of hydrogel matrices prepared using Bipol, Hypol Hydrogel and Hypol 2002 prepolymers and following the typical processes described in Examples 5 and 6, was measured over time using a swelling medium comprising pH 4.0 buffer with 5 mg/ml sodium bisulfite and 5 mg/ml citric acid in deionized water. Results are tabulated in Table V. Weight is given in milligrams. Swelling ratio, weight divided by dry weight, is in parenthesis. Figure 2 depicts the swelling behavior over time of hydrogel matrices using the values in Table V.

TABLE V

## Swelling Behavior Over Time

| Pre-polymer | 0 min | 30 min | 60 min | 100 min | 150 min | 16 hr | 40 hr | dry |
|---|---|---|---|---|---|---|---|---|
| Biopol | 106 mg (1.68) | 251 mg (3.98) | 329 mg (5.22) | 397 mg (6.30) | 460 mg (7.30) | 858 mg (13.62) | 1018 mg (16.16) | 63 mg |
| Hypol Hydrogel | 95 mg (1.12) | 226 mg (2.66) | 301 mg (3.54) | 365 mg (4.29) | 419 mg (4.93) | 598 mg (7.04) | 637 mg (7.49) | 85 mg |
| Hypol 2002 | 39 mg (1.86) | 55 mg (2.62) | 60 mg (2.86) | 63 mg (3.00) | 66 mg (3.14) | 78 mg (3.71) | 78 mg (3.71) | 21 mg |

EP 0 524 718 A1

## EXAMPLE 8

Incorporation of a Drug Solution in the Hydrogel Matrix-Loading Kinetics

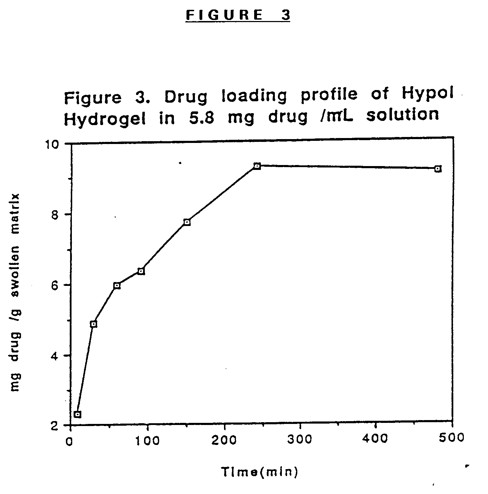

A solution of Arbutamine™ [1] as 5.8 mg/ml with 1mg/ml bisulfite, 2.1 mg/ml citric acid adjusted to pH 4 was used to study loading kinetics of a hydrogel matrix of the present invention. The matrix was incubated in this solution and the amount of drug in the matrix was assayed at certain times. Figure 3 depicts a loading profile for the drug in matrix over time.

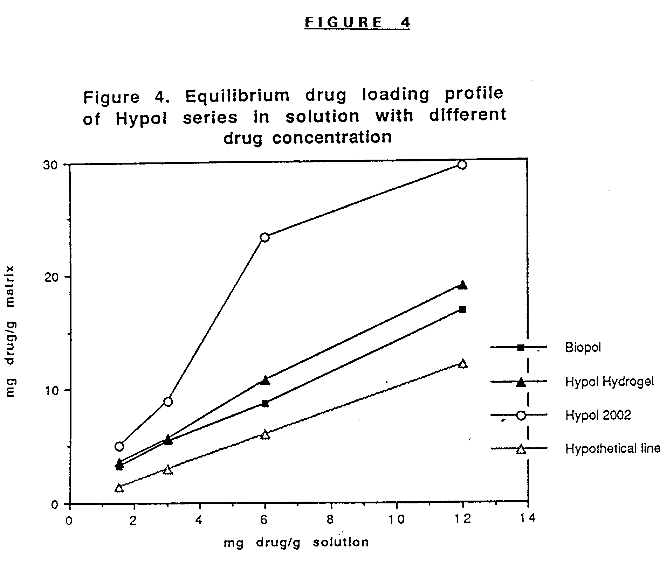

According to Figure 3, it takes about four hours for a Hypol Hydrogel matrix fully swollen with deionized water to completely equilibrate with the loading solution in terms of drug concentration. An interesting observation from this study is that the drug was incorporated into the swelled matrix at a concentration of 9 mg/g, where concentration of drug in the loading solution was 5.8 mg/ml. This difference was thought to be due to van der Waals interaction between drug and soft segment of polymer chain. Correlation between amount of drug present in loading solution and amount present in matrix for hydrogel matrices prepared using Hypol 2002, Hypol Hydrogel and Biopol prepolymers is depicted in Figure 4. The "hypothetical line" (in Figure 4) is also drawn for comparison, after assuming that the drug concentrations in loading solution and matrix are the same.

## EXAMPLE 9

Swelling Behavior of Hydrogel Matrices

Hydrogel matrices prepared according to Examples 1 to 3 using the below-noted amounts of prepolymer, first and second solvents were swollen using an aqueous solution containing 1 mg/ml sodium bisulfite and 2.1 mg/ml citric acid, pH 4.0. Table VI reports the swelling ratio after 24 hours.

[1]   Arbutamine™   (GP2-121-3)   is   1-(3,4-dihydroxyphenyl)-2-(4-(4-hydroxyphenyl)butylamino)ethanol hydrochloride.

## TABLE VI

### Swelling Dependency on Initial Prepolymer Content

| Prepolymer Amount | First Solvent | Second Solvent | Swelling Ratio After 24 Hours |
|---|---|---|---|
| Hypol Hydrogel, 0.3 g | 2 ml acetone | 1 ml water/ 1 ml acetone | 9.7 |
| Hypol Hydrogel, 0.4 g | 2 ml acetone | 1 ml water/ 1 ml acetone | 9.8 |
| Hypol Hydrogel, 0.5 g | 2 ml acetone | 1 ml water/ 1 ml acetone | 7.5 |
| Hypol Hydrogel, 0.6 g | 2 ml acetone | 1 ml water/ 1 ml acetone | 7.5 |
| Hypol Hydrogel, 0.3 g | 2 ml actone | 1.5 ml water/ 0.5 ml acetone | 9.5 |
| Hypol Hydrogel, 0.4 g | 2 ml acetone | 1.5 ml water/ 0.5 ml acetone | 8.4 |
| Hypol Hydrogel, 0.5 g | 2 ml acetone | 1.5 ml water/ 0.5 ml acetone | 7.9 |
| Hypol Hydrogel, 0.6 g | 2 ml acetone | 1.5 ml water/ 0.5 ml acetone | 7.0 |
| Hypol 2002, 0.4 g | 2 ml acetone | 1 ml water/ 1 ml acetone | 3.5 |
| Hypol 2002, 0.5 g | 2 ml acetone | 1 ml water/ 1 ml acetone | 3.0 |
| Hypol 2002, 0.6 g | 2 ml acetone | 1 ml water/ 1 ml acetone | 2.7 |
| Hypol 2002, 0.8 g | 2 ml acetone | 1 ml water/ 1 ml acetone | 2.4 |
| Biopol, 0.3 g | 2 ml acetone | 2 ml water/ 2 ml acetone | 19 |
| Biopol, 1.0 g | 2 ml acetone | 1 ml water/ 1 ml acetone | 9 |

EP 0 524 718 A1

EXAMPLE 10

Swelling Behavior of Hydrogel Matrices

Hydrogel matrices prepared using Hypol Hydrogel prepolymer prepared according to Example 5 and Hypol 2002 prepolymer prepared according to Example 6 were swollen using aqueous media, pH 4.0, comprising 1 mg/ml sodium bisulfite, 2.1 mg/ml citric acid and the noted amount of propylene glycol. Swelling ratios were determined after 24 hours at the indicated temperature and are reported in Table VII. R.T. indicates room (ambient) temperature.

## TABLE VII

### Swelling Ratio Temperature Dependency.

| % Propylene Glycol | Temp (°C) | Swelling Ratio | |
|---|---|---|---|
| | | Hypol Hydrogel | Hypol 2002 |
| 0 | 4 | 11.2 | ---- |
| | R.T. | 8.4 | 3.8 |
| | 37 | 6.5 | ---- |
| 30 | 4 | ---- | ---- |
| | R.T. | 10.8 | 4.8 |
| | 37 | 10.3 | 4.2 |
| 40 | 4 | ---- | ---- |
| | R.T. | 11.1 | 4.8 |
| | 37 | 11.7 | 4.7 |
| 50 | 4 | 12.1 | ---- |
| | R.T. | 11.4 | 5.2 |
| | 37 | 15.3 | ---- |

EXAMPLE 11

Effect of Propylene Glycol on Dehydration

Hydrogel matrices were prepared using Hypol Hydrogel prepolymer according to Example 5. The matrices were swollen in an aqueous medium pH 4.0 comprising 1 mg/ml sodium bisulfite, 2.1 mg/ml citric acid, either 0% or 50% propylene glycol and deionized water. The matrices were allowed to swell for 24 hours. The matrices were then placed in an open beaker and the weight change of swollen matrix over time was monitored. Results are tabulated in Table VIII.

## TABLE VIII

### Effect of Propylene Glycol in Swelling Media on Dehydration

| Time (hours) | 50% Propylene Glycol | No Propylene Glycol |
|---|---|---|
| 0 | 392 | 368 |
| 1 | 373 | 340 |
| 2 | 357 | 311 |
| 5 | 321 | 240 |
| 24 | 268 | 46 |

### EXAMPLE 12

#### Drug Stability

Stability of Arbutamine in electrode pads using the hydrogel matrix prepared from Hypol Hydrogel prepolymer was studied using the 3M Red Dot™ Electrode Construction. (The 3M Red Dot™ electrode is manufactured by 3M's medical-surgical division and used primarily for ECG monitoring. It is composed of a soft cloth backing, Sureseal Cap Seal, and the gel. The 3M Sureseal Cap Seal minimizes the dehydration of the gel.)

The drug-formulated hydrogel matrix was prepared by diffusional exchange in solution at pH 4.0 with 45 mg or 90 mg of Arbutamine™ (GP 2-121-3), 21 mg of citric acid, and 10 mg of sodium bisulfite in 10 ml of deionized water. The matrix incorporating the drug was placed in the 3M Red Dot electrode (after removing the gel supplied with the electrode). The electrode was secured in an aluminum pouch with or without a nitrogen flush (as noted). Drug stability studies were carried out at room temperature and 4°C. (A study at 50°C was terminated due to severe dehydration). Data obtained by isocratic HPLC assay showed no significant drug degradation after six months (see Table IX). The drug stability in the sample electrodes stored under nitrogen did not appear different. It is believed that the increase in drug concentration over time, as assayed by HPLC, was due to dehydration of the hydrogel.

TABLE IX. Drug Stability in Hypol Hydrogel Matrix over Time.

| Sample No. | Temperature | Atmosphere | 0 weeks | 1 weeks | 2 weeks | 4 weeks | 8 weeks | 6 months |
|---|---|---|---|---|---|---|---|---|
| 1 | Room | Nitrogen | 12.5 | 12.4 | 12.8 | 13.3 | 13.8 | 14.9 |
| 2 | Room | Nitrogen | 13.4 | 13.2 | 13.0 | 14.0 | 14.1 | 15.8 |
| 3 | 4°C | Nitrogen | 12.2 | 13.3 | 13.3 | 13.2 | 13.8 | 14.6 |
| 4 | 4°C | Nitrogen | 12.7 | 13.5 | 13.0 | 13.3 | 13.8 | 14.5 |
| 5 | Room | Air | 9.1 | 9.3 | 9.5 | 9.6 | 10.1 | 10.7 |
| 6 | Room | Air | 9.0 | 9.3 | 9.5 | 9.5 | 10.0 | 10.6 |

EP 0 524 718 A1

EXAMPLE 13

Drug Release from Hydrogel Matrix

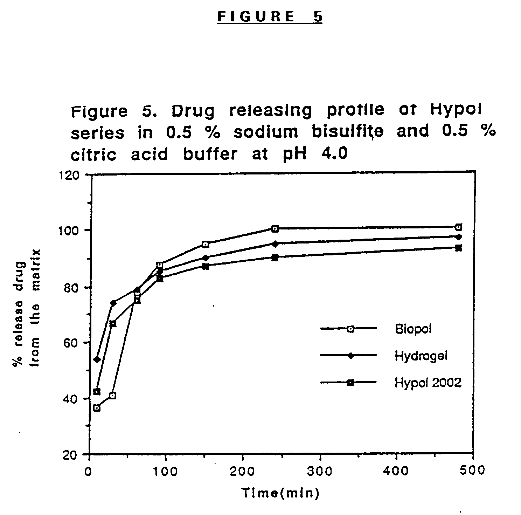

Profiles of release of drug in an aqueous solution of 0.5% sodium bisulfite and 0.5% citric acid buffer at pH 4.0 were made using hydrogel matrices prepared from Hypol Hydrogel, Hypol 2002 and Biopol prepolymers. According to these studies, more than 85% of the drug was released into solution within 3 hours from all three matrices. Data are set forth in Figure 5.

EXAMPLE 14

Iontophoretic Function - In Vitro Studies

Iontophoretic function of a hydrogel matrix prepared using Hypol Hydrogel prepolymer was studied in vitro using a commercially available Franz-type transport cell. The drug-loaded hydrogel was placed in the reservoir of a well type electrode. The upper (donor) portion of the cell was separated from the buffer filled bottom (receptor) portion by a layer of porcine skin or a proprietary synthetic membrane. In a typical protocol, current is applied to the anode, this drives the positively charged drug through the rate determining membrane into the receptor solution. The amount of drug in the receptor solution is assayed using HPLC.

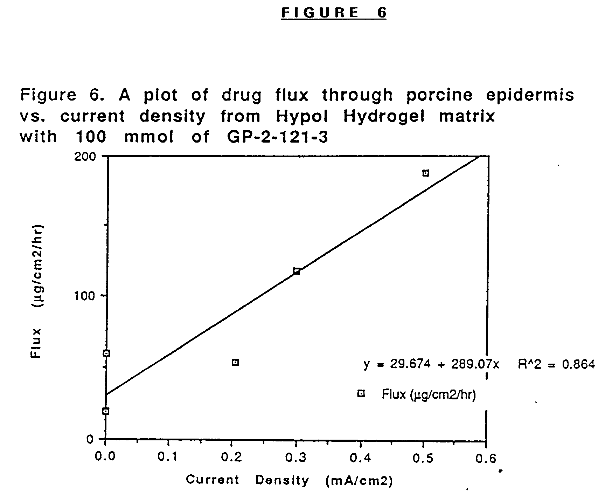

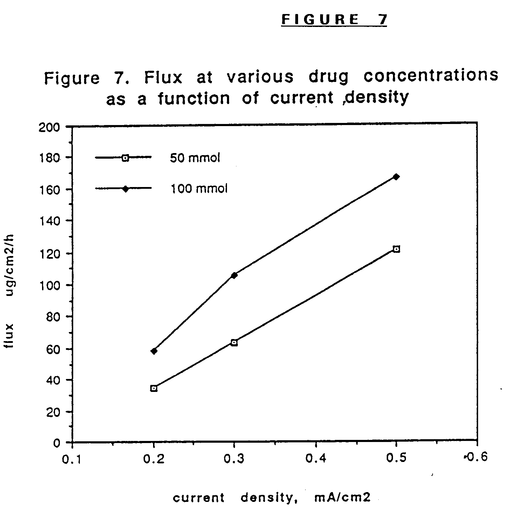

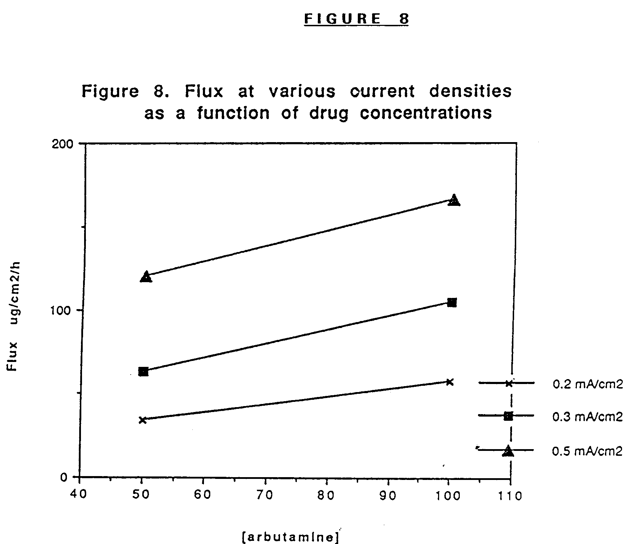

Figure 6 depicts the results of a study done using a Hypol Hydrogel matrix filled with drug solution at 100 mM and measuring drug flux through porcine epidermis versus current density. The measured response indicated a normalized flux of about 290 μg/mA/hr. Figure 7 depicts drug flux data from several studies using varying concentrations of GP 2-121-3 as a function of current density. Figure 8 depicts drug flux data as a function of a drug concentration at various current densitites.

As an alternative for porcine epidermis, flux studies were performed using a synthetic membrane which consisted of a hydrophobic polyurethane main backing polymer, Tecoflex 80A (Thermedics, Inc.) and a hydrophilic embedding or completing interpenetrating network ("IPN") material, polyethylene glycol as components. The synthetic membrane was composed of 33% polyethylene glycol (M.W. 3350) and 67% Tecoflex 80A. Thickness of the membrane was controlled between 3 mls to 4 mls by solvent casting. Flux data obtained using this synthetic membrane is reported in Table X.

## TABLE X

### Drug Flux at 0.5 mA/cm$^2$ and Slope of Plot Between Flux and Current Density Using Synthetic Membrane

| Run # | Drug Flux at 0.5mA/cm$^2$ | Slope of Plot Between Flux and Current Density |
|---|---|---|
| 1 | 156.4 | 276 |
| 2 | 149.8 | 286 |
| 3 | 133.9 | 249 |
| 4 | 153.1 | 277 |
| 5 | 138.8 | 253 |
| 6 | 91.4 | 147 |
| Methocel | 179.0 | 358.1 |
| Mean (S.D.) | 137.2 (13.4) | 248 (29) |

## EXAMPLE 15

### Iontophoretic Function - In Vivo Studies

In vivo animal tests were performed using labrador dogs about 3 years old and about 27 kg weight to demonstrate iontophoretic drug delivery using these hydrogel matrices. Three dogs (BO, BA and PO) were used over 13 experiments. The experiments measured heart rate response on current application and dose response using hydrogel matrices (prepared using Hypol Hydrogel prepolymer) with different drug concentrations. The experimental protocol involved delivery of the appropriate drug concentration at a $1.0 \text{ mA/cm}^2$ current density over a ten minute time period. Prior to drug delivery, the table-trained dogs' right or left hind flank was shaved and cleaned with soap and crater in preparation for drug and in-different electrodes. Three areas on the back were also shaved and cleansed for ECG leads. Upon electrode placement, a stable baseline heart rate was established for transdermal iontophoretic administration. The heart rate response to the drug delivery was monitored for 45 minutes. Results are set forth in Table XI for each dose in terms of heart rate, delay time upon onset of current, and estimated half-life of decline of heart rate. For comparison results from a study using a hydroxypropyl methylcellulose formulation (methocel) are given. Table XI indicates that use of the hydrogel matrices gave increased heart rates at lower drug concentrations (of arbutamine) than using methocel.

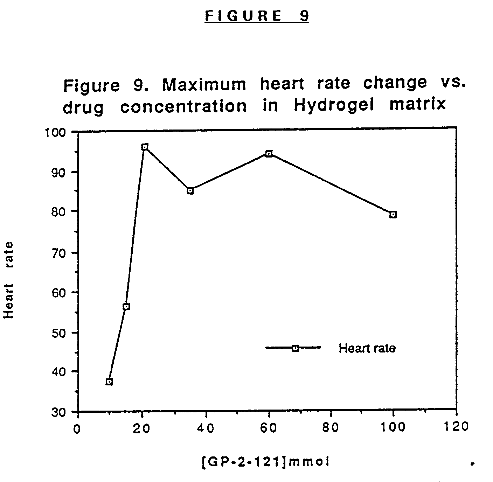

Figure 9 depicts a plot of maximum heart rate change versus drug concentration using a Hypol Hydrogel matrix.

## TABLE XI

### Heart Rate Response for Various Drug Concentrations
Using Hypol Hydrogel Matrix or Methocel

| Gel | Drug mmol | Heart Rate (bpm) | Delay Time (min) | Half-Life (Min.) |
|---|---|---|---|---|
| Hypol Hydrogel | 10 | 37.5 (1.0) | 1.0 (1.0) | 7.3 (0.7) |
| " | 15 | 56.3 (23.6) | 2.4 (2.5) | 8.9 (4.9) |
| " | 21 | 95.9 (4.6) | 2.5 (1.6) | 40.0 (34.7) |
| " | 35 | 84.7 (21.1) | 5.7 (10.2) | 31.7 (25.8) |
| " | 60 | 94.0 | 3.9 | 32.2 |
| " | 100 | 78.4 | 6.1 | 57.7 |
| Methocel | 48 | 77.3 (6.4) | 2.3 (0.5) | 35.8 (5.2) |

Note: Numbers in parentheses are standard deviations for 2 runs for 10 mmol and 21 m mol, 3 runs for 15 mmol, 4 runs and 7 runs for 48 mmol (methocel).

EP 0 524 718 A1

**Claims**

1.  A method of preparing a polyurethane hydrogel matrix suitable for use in transdermal iontophoretic drug delivery which comprises:

    (a) dissolving an isocyanate capped prepolymer in a first solvent which comprises an anhydrous aprotic water-miscible organic solvent to give a prepolymer solution;

    (b) mixing the prepolymer solution of step (a) with a second solvent which comprises water and optionally a water miscible organic solvent to give a hydrogel forming mixture.

    (c) allowing the mixture of step (b) to cure to give a hydrogel matrix;

    wherein the ratio of prepolymer to water is such that the ratio of the isocyanate content of the prepolymer to amount of water is from about 0.04 meq isocyanate to about 4 meq isocyanate per ml water and the ratio of isocyanate content of the prepolymer to combined volume of first and second solvent is from about 0.01 meq to about 1 meq isocyanate per ml combined solvent.

2.  A method according to claim 1 wherein said first solvent comprises acetone.

3.  A method according to claim 2 wherein said water miscible organic solvent comprises acetone.

4.  A method according to any one of claims 1 to 3 wherein the prepolymer comprises from about 0.4 to about 1.6 meq/g isocyanate.

5.  A method according to any one of claims 1 to 4 wherein said prepolymer comprises a polyisocyanate terminated polyether derived from toluene diisocyanate.

6.  A method according to any one of claims 1 to 4 wherein said prepolymer comprises a polyisocyanate terminated polyether derived from aliphatic diisocyanate.

7.  A method according to any one of the preceding claims further comprising:

    (d) swelling the hydrogel matrix from step (c) in an aqueous swelling medium.

8.  A method according to claim 7 wherein said aqueous swelling medium comprises at least about 30% propylene glycol.

9.  A method according to claim 7 or 8 wherein said aqueous swelling medium comprises 1-(3,4-dihydroxyphenyl)-2-(4-(4-hydroxyphenyl)butylamino)ethanol hydrochloride.

10. A method of preparing a polyurethane hydrogel matrix suitable for use in transdermal iontophoretic drug delivery which comprises:

    (a) dissolving an isocyanate capped prepolymer in a total solvent which comprises water and a water miscible organic solvent to give a hydrogel-forming solution; and

    (b) allowing the mixture of step (a) to cure to give a hydrogel matrix;

    wherein the ratio of prepolymer to water is such that the ratio of the isocyanate content of the prepolymer to the amount of water is from about 0.04 meq isocyanate to about 4 meq isocyanate and the ratio of isocyanate content of the prepolymer to amount of total solvent is from about 0.01 meq to about 1 meq isocyanate per ml total solvent, and total solvent comprises at least one part of water miscible organic solvent per part water.

11. A method according to claim 10 wherein total solvent comprises from about 0.2 parts to about 1 part water per part water miscible organic solvent.

12. A method according to claim 10 or 11 wherein the hydrogel forming solution comprises from about 5% to about 25% prepolymer.

13. A method according to any one of claims 10 to 12 wherein said water miscible organic solvent is acetone.

14. A method of preparing a polyurethane hydrogel matrix suitable for use in transdermal iontophoretic drug delivery which comprises:

    (a) preparing a hydrogel forming mixture which comprises an isocyanate-capped prepolymer in total solvent which comprises water and a water miscible organic solvent wherein the ratio of isocyanate content of the prepolymer to water is from about 0.04 meq to about 4 meq isocyanate per ml water and the ratio of isocyanate content to total solvent is from about 0.01 meq to about 1 meq isocyanate per ml total solvent and total solvent comprises about 0.2 parts to about 1 part water per part water miscible organic solvent; and (b) curing the hydrogel forming mixture to give a hydrogel matrix.

15. A method according to claim 14 wherein said hydrogel forming solution comprises from about 5% to about 25% prepolymer.

16. A method according to claim 14 or 15 wherein said water miscible organic solvent comprises acetone.

17. A method according to any one of claims 14 to 16 further comprising
    (c) swelling the hydrogel matrix from step (b) in an aqueous swelling medium.

18. A method according to claim 17 wherein said swelling medium comprises at least about 30% propylene glycol.

19. A method according to claim 17 or 18 wherein said hydrogel matrix is swollen at a temperature greater than its anticipated temperature for storage and use.

20. A hydrogel matrix prepared by a method of any claims 1, 3, 4, 5, 6, 7, 8, 10, 14, 17 or 19.

21. A hydrogel matrix which is derived from a polyisocyanate polyether prepolymer and which is substantially optically transparent and has a swelling ratio of at least about 4.

22. A hydrogel matrix according to claim 21 having a swelling ratio of about 7 to about 15.

23. A method according to claim 21 or 22 swelled in a swelling medium which comprises at least about 30% propylene glycol.

24. A hydrogel matrix according to any one of claims 21 to 23 wherein said prepolymer is Hypol hydrogel (a polyurethane having terminal isocyanate groups and having an average molecular weight of from 100 to 200,000.

25. A hydrogel matrix according to any one of claims 21 to 23 wherein said prepolymer is Hypol hydrogel, Hypol 20002 or Biopol (a polyurethane having terminal isocyanate groups and having an average molecular weight of 100 to 200,000 and an isocyanate content of about 0.8 meq/g or 1.6 meq/g or an isocyanate capped polyoxyalkylene polyol having an isocyanate content of about 0.4 meq/g).

Figure 1. Swelling profile of Hypol Hydrogel in aqueous buffer with different ionic strength at pH 5.0

F I G U R E   2

Figure 2. Swelling profile of Hypol series in 0.5 % sodium bisulfite and 0.5 % citric acid buffer at pH 4.0

FIGURE 3

Figure 3. Drug loading profile of Hypol Hydrogel in 5.8 mg drug /mL solution

EP 0 524 718 A1

**F I G U R E  4**

Figure 4. Equilibrium drug loading profile of Hypol series in solution with different drug concentration

**F I G U R E   5**

Figure 5. Drug releasing profile of Hypol series in 0.5 % sodium bisulfite and 0.5 % citric acid buffer at pH 4.0

**F I G U R E   6**

Figure 6. A plot of drug flux through porcine epidermis
vs. current density from Hypol Hydrogel matrix
with 100 mmol of GP-2-121-3

y = 29.674 + 289.07x    R^2 = 0.864

□    Flux (µg/cm2/hr)

**F I G U R E   7**

Figure 7. Flux at various drug concentrations
as a function of current density

<u>**F I G U R E   8**</u>

Figure 8. Flux at various current densities
as a function of drug concentrations

FIGURE 9

Figure 9. Maximum heart rate change vs.
drug concentration in Hydrogel matrix

**F I G U R E  10**

Figure 10. Swelling behavior of Hypol
Hydrogel at different temperatures

FIGURE  11

Figure  11.  Temperature  dependancy  of  Hypol
Hydrogel  swelling  in  various  solutions

European Patent
Office

# EUROPEAN SEARCH REPORT

Application Number

EP 92 30 4602

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| P,X | EP-A-0 454 930 (MINNESOTA MINING AND MANUFACTURING)<br>* page 3, line 49 - page 5, line 8; claims 1-3; example 3 *<br>--- | 1-7 | C08G18/08<br>C08G18/10<br>A61K9/00<br>C08G18/76 |
| A | EP-A-0 333 899 (W.R. GRACE)<br>* page 6, line 1 - page 8, line 17; claims 1-6 *<br>--- | 1,2 | |
| A | US-A-4 315 703 (GASPER)<br>* column 4, line 5 - line 39; claim 1 *<br>----- | 1 | |
| | | | **TECHNICAL FIELDS SEARCHED (Int. Cl.5 )**<br><br>C08G<br>A61N |

The present search report has been drawn up for all claims

| Place of search | Date of completion of the search | Examiner |
|---|---|---|
| THE HAGUE | 15 OCTOBER 1992 | BOURGONJE A.F. |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P0401)